# EUROPÄISCHE PATENTSCHRIFT

(11) **EP 0 618 006 B1**
(45) Veröffentlichungstag und Bekanntmachung des Hinweises auf die Patenterteilung: **13.11.1996**
(21) Anmeldenummer: 94104377.0
(22) Anmeldetag: 19.03.1994
(51) Int. Cl.: B01J 23/755, C07C 29/141

(54) **Hydrierkatalysator, ein Verfahren zur Herstellung und Verwendung**
Hydrogenation catalyst, process for its preparation and its use
Catalyseur d'hydrogénation, procédé pour sa préparation et son utilisation

(30) Priorität: 27.03.1993 DE 4310053
(43) Veröffentlichungstag der Anmeldung: 05.10.1994
(73) Patentinhaber: HOECHST AKTIENGESELLSCHAFT, 65926 Frankfurt am Main (DE)
(72) Erfinder: Deckers, Gregor, Dr. Dipl.-Chem., D-46509 Xanten (DE); Diekhaus, Gerhard, Dr. Dipl.-Chem., D-46147 Oberhausen (DE); Dorsch, Bernd, Dr. Dipl.-Chem., D-46244 Bottrop (DE); Frohning, Carl Dieter, Dr. Dipl.-Chem., D-46485 Wesel (DE); Horn, Gerhardt, Dr. Dipl.-Chem., D-46147 Oberhausen (DE); Horrig, Horst Burkhard, D-46147 Oberhausen (DE)

(56) Entgegenhaltungen:
- EP-A- 0 114 704
- EP-A- 0 340 848
- EP-A- 0 389 158
- EP-A- 0 398 668
- EP-A- 0 470 344
- EP-A- 0 472 918
- US-A- 3 868 332

## Beschreibung

Die vorliegende Erfindung betrifft einen reduzierten Hydrierkatalysator, welcher aus Nickel, Nickeloxid, Magnesiumoxid und einem wasserunlöslichen Trägermaterial besteht.

Katalysatoren auf der Basis von Nickel als aktiver Katalysatorbestandteil für die Hydrierung von Aldehyden gehören zum Stand der Technik.

Im EP-A-0 322 049 wird ein Hydrierkatalysator, bestehend aus
1) einem molaren Verhältnis von SiO₂/Ni = 0,15 - 0,35
2) einem molaren Verhältnis von (Mg; Ba)/Ni = 0 - 0,15
beschrieben, bei welchem ein Teil des Nickels in metallischer Form vorliegt.

Ein weiterer Hydrierkatalysator mit 60 Gew.-% Ni, 1,6 Gew.-% Mg (gerechnet als MgO) und 36 Gew.-% Kieselgur als Trägermaterial, entsprechend 35,2 g Kieselgur je Mol Ni ist aus EP-A-0 472 918 bekannt.

Die bekannten Hydrierkatalysatoren auf Basis Nickel können bei der Hydrierung von Aldehyden nur bei Hydrierungstemperaturen bis etwa 1oo °C eingesetzt werden, da mit steigender Hydrierungstemperatur unerwünschte Nebenprodukte gebildet werden, welche teilweise nur durch eine aufwendige Destillation abgetrennt werden können.

Es war daher die Aufgabe gestellt, einen Hydrierkatalyator zur Verfügung zu stellen, der bei hohen Durchsatzgeschwindigkeiten und Hydrierungstemperaturen von oberhalb 11o °C eine hohe Selektivität und Umsatzgrade von mehr als 99,5 % aufweist.

Überraschend konnte diese Aufgabe mit einem Hydrierkatalysator gelöst werden, der aus
- 25 bis 5o: Gewichts% Nickel (metallisch)
- 1o bis 35: Gewichts% Nickeloxid
- 4 bis 12: Gewichts% Magnesiumoxid
- 1 bis 5: Gewichts% Natriumoxid
- Rest: Trägermaterial
besteht und die Summe aus Nickel und Nickeloxid 4o bis 7o Gewichts% beträgt, wobei der Hydrierkatalysator eine, nach BET bestimmte, Oberfläche von 8o bis 2oo m²/g und ein, durch Hg-Porosimetrie bestimmtes, Gesamtporenvolumen von 0,35 bis 0,6 ml/g hat, und das Gesamtporenvolumen aufgeteilt ist in
- 3o bis 6o: Volumenprozente mit einem Porenradius von ≦ 4 nm (4o Å),
- 4 bis 1o: Volumenprozente mit einem Porenradius von > 4 bis 30 nm (4o bis 3oo Å),
- 3o bis 6o: Volumenprozente mit einem Porenradius von > 30 bis 500 nm (3oo bis 5ooo Å).

Der erfindungsgemäße Hydrierkatalysator kann weiterhin wahlweise und bevorzugt dadurch gekennzeichnet sein,
a) daß 5 bis 1o Atomlagen auf der Oberfläche des Hydrierkatalysators, bestimmt nach der SAM-Untersuchung,
   - 18 bis 3o,: vorzugsweise 2o bis 28 Atom% Ni
   - 1,2 bis 3,o,: vorzugsweise 1,5 bis 2,5 Atom% Na
   - 2,8 bis 4,8,: vorzugsweise 3,2 bis 4,5 Atom% Mg
   enthalten;
b) daß die metallische Nickeloberfläche eine, durch Chemiesorption von Wasserstoff bestimmte, Größe von 100 bis 130 m²/g Ni hat;
c) daß er als Tragermaterial Aluminiumoxid oder Siliciumdioxid, insbesondere in Form von Kieselsäure, Kieselgel, Kieselgur oder Kieselerde enthält.

Die Erfindung betrifft weiterhin ein Verfahren zur Herstellung des Hydrierkatalysators, welches dadurch gekennzeichnet ist, daß man in einer Fällstufe einen Grünkatalysator aus Nickelsalz, Magnesiumsalz und Soda sowie Trägermaterial herstellt, diesen Grünkatalysator nach Abtrennen der Mutterlauge und partieller Waschung in Alkalilösung aufschlämmt, von der Flüssigphase abtrennt, trocknet und den getrockneten Grünkatalysator mit Wasserstoff behandelt, bis 42 bis 83 Gew% des gesamten Nickelanteils in metallischer Form vorliegen.

Eine wahlweise und bevorzugte Verfahrensdurchführung ist gegeben, wenn man
d) in der Fällstufe in eine 95 bis 100°C heiße 0,9 bis 1,1 molare Sodalösung eine 95 bis 100°C heiße wäßrige Lösung, enthaltend 0,5 bis 0,8 mol/l Nickelsalz und 0,1 bis 0,2 mol/l Magnesiumsalz, bis zu einem molaren Verhältnis von Na₂CO₃ : (Ni + Mg) = 1 : 0,60 bis 0,65 einrührt und anschließend umgehend innerhalb von 0,5 bis 5 Minuten das Trägermaterial zusetzt und den gebildeten Grünkatalysator von der Mutterlauge abfiltriert, mit heißem Wasser partiell wäscht, bis das ablaufende Waschwasser eine Leitfähigkeit von 1500 bis 2000 µS hat; danach den Grünkatalysator in einer 1- bis 3-fachen Wassermenge suspendiert und mit 0,06 bis 0,08 Mol Natronlauge oder 0,03 bis 0,04 Mol Soda pro Mol in der Fällstufe eingesetzten Nickelmenge verrührt und nach 1 bis 5 Stunden Rührzeit bei einer Temperatur von 4o bis 6o °C den Grünkatalysator aus der Suspension abtrennt;
e) den aus der Suspension abgetrennten und getrockneten Grünkatalysator bei einer Temperatur von 35o bis 45o °C reduziert, indem man den getrockneten Grünkatalysator mit o,5 bis 5,o Nm³/h Reduktionsgas pro kg Grünkatalysator, wobei das Reduktionsgas 80 bis 100 Vol% Wasserstoff enthält, behandelt.
f) 1 bis 3 Nm³/h Reduktionsgas pro kg getrockneten Grünkatalysator einsetzt.

Zur besseren Formgebung kann man dem Hydrierkatalysator o,5 bis 5 Gewichts% Graphit zusetzen.

Der erfindungsgemäße Hydrierkatalysator kann zur Hydrierung von Propanal und n-Butanal und i-Butanal, vorzugsweise bei 11o bis 16o °C verwendet werden.

Mit dem erfindungsgemäßen Hydrierkatalysator ist die Hydrierung von Propanal und Butanal unter gleichzeitiger Gewinnung von Wasserdampf mit Überdruck zur Verbesserung der Wirtschaftlichkeit der Hydrierung durchführbar. Gegenüber üblichen Hydrierkatalysatoren kann die Hydrierung bei erhöhter Katalysatorbelastung von 0,8 bis 1,0 kg/h Aldehyd pro kg Katalysator durchgeführt werden.

Die Selektivität ist größer als 99,5 %, meist größer als 99,9 %; etwa 0,01 % des eingesetzten Aldehyds verbleiben im Fertigprodukt. Weniger als 0,1 % des Aldehyds werden in Kohlenmonoxid, Ether, Acetale und Ester umgewandelt.

Bei der Hydrierung von n-Butanal liegt die Bildung von Dibutylether unter 5o ppm, wodurch eine Destillation des Fertigproduktes entfallen kann.

Bei der Hydrierung von n-Propanal liegt die Bildung von Dipropylether unter 2o ppm.

Angewandte Untersuchungsmethoden
1. BET-Oberflächen-Bestimmung
Das Verfahren zur Bestimmung der BET-Gesamtoberfläche nach Brunauer, Emmett und Teller ist in J. Amer. Chem. Soc. 6o (1938), Seite 3o9, beschrieben.
2. Porenvolumen-Bestimmung durch Hg-Porosimetrie (Gesamtporenvolumen und Porenverteilung)
Das Verfahren zur Porenvolumen-Bestimmung durch Hg-Porosimetrie bis 39oo bar nach H.L. Ritter, L.C. Drake ist in Ind. Engng. chem. analyt. Edit, 17 (1945) 782 beschrieben.
3. Oberflächen-Bestimmung durch Chemiesorption
Das Verfahren zur Bestimmung der Nickeloberfläche durch Chemiesorption der bei 2o °C aufgenommenen Wasserstoffmenge ist im J. of Catalysis 81 (1983) 2o4 und 96 (1985) 517 beschrieben.
4. Porenradius-Bestimmung
Das Verfahren zur Porenradius-Bestimmung ist von S.J. Gregg, K.S.W. Sing, Adsorption Surface Area and Porosity, Academic Press New York-London (1967), Seiten 16o bis 182 beschrieben.
5. Oberflächenuntersuchung mit der SAM-Spektroskopie (Scanning Auger Microprobe)
Die Untersuchungen erfolgten mit dem SAM-Spektrometer PHI 66o der Firma Perkin-Elmer.
Die zu untersuchenden Proben wurden in einer Probenkammer mit Hilfe einer Turbomolekularpumpe auf ein Vakuum von ≦ 1,33 µPa (≦ 1 x 1o ⁻⁸ Torr) evakuiert.
Eine Elektronenkanone lieierte den Elektronenstrahl, mit dem die Probe beschossen wurde. Aufgrund starker Aufladung der Proben durch den Elektronenstrom wurden sogenannte MULTIPLEX-Messungen an 5 verschiedenen Stellen jeder Probe durchgeführt, wobei nur die Energiebereiche der zu erwartenden Elemente abgetastet wurden, um eine möglichst kurze Meßzeit zu erreichen.
Die SAM-Untersuchungs-Methode ist beschrieben in "Practical surface analysis by Auger and x-ray photoelectronic spectroscopy" von D. Briggs and M. Seah, Verlag John Wiley and Sons, New York, London (1983), Seiten 217 ff und 283 ff.
Die Messung und Analyse der von der Probe emittierten AUGER-Elektronen erfolgte mit Hilfe eines zylindrischen Spiegelanalysators.
Bei den Untersuchungen wurden folgende Bedingungen gewählt:

| | |
|---|---|
| Energie-Auflösung (E/E): | 0,6 % |
| Anregungsenergie: | 10 k V/10 m A |
| Laterale Auflösung: | ca. 22o nm |

Der quantitativen Auswertung wurden die Empfindlichkeitsfaktoren der reinen Elemente, die im "Handbook of Auger Spectroscopy" veröffentlicht sind, zugrundegelegt.
Gemessen und ausgewertet wurden die entsprechenden Werte der Elemente Nickel, Natrium und Magnesium.

### Hydrierkatalysator-Eigenschaften

Die speziellen physikalischen und chemischen Eigenschaften des Hydrierkatalysators, die letztendlich die Voraussetzung für sein vorteilhaftes Hydrierverhalten sind, werden im wesentlichen durch folgende Merkmale und Maßnahmen bei der Herstellung erreicht.

In der Fällstufe erfolgt die gemeinsame Fällung von basischem Nickel-Magnesium-Mischcarbonat und dessen Anlagerung an das Trägermaterial. Die Fällungsbedingungen sind so gewählt, daß der aus basischem Mg-Carbonat bestehende Anteil des Niederschlages in möglichst schwerlöslicher Form vorliegt.

Der in der Fällstufe erhaltene Grünkatalysator wird erfindungsgemäß nur partiell soweit gewaschen, daß wenig ausgefälltes basisches Magnesiumcarbonat aus dem Grünkatalysator ausgewaschen wird.

Zur Erfindung gehört die gezielte Nachalkalisierung des partiell gewaschenen Grünkatalysators, wodurch die erforderliche Anreicherung von Alkali auf der Katalysatoroberfläche erreicht wird.

Die Trocknung des Grünkatalysators ist nicht kritisch. Sie kann in einem relativ weiten Bereich von Trocknungsbedingungen beispielsweise im Luftstrom bei 5o bis 1oo °C durchgeführt werden.

Kritisch und erfinderisch ist die Reduktion des Grünkatalysators zum Hydrierkatalysator. Die Reduktion ist so durchzuführen, daß unter Einhaltung der Temperatur von 35o bis 45o °C und einer Wasserstoffströmungsgeschwindigkeit von 0,25 bis 0,75 m/s nur eine teilweise Reduktion der nickelhaltigen Komponente erfolgt. Ein Reduktionsgrad von 42 bis 83 % hat sich als vorteilhaft erwiesen.

Bei der Anwendung in Festbetthydrierungen wird der Grünkatalysator vor der Trocknung z.B. zu Extrudat verformt, getrocknet, reduziert und in dieser Form eingesetzt oder nach der Reduktion durch Behandlung mit geringen Mengen O₂ in N₂ in bekannter Weise stabilisiert, (H. Blume, W. Naundorf und A. Wrubel, Chem. Techn., Bd. 15 (1963), Seite 583).

Es wurde gefunden, daß eine Verarmung der Oberfläche des Hydrierkatalysators an Na eine Zunahme von Spalt- und Nebenreaktionen verursacht, die zum Verlust von Wertprodukten führen.

Bei übermäßigen Na-Konzentrationen an der Oberfläche des Hydrierkatalysators nimmt die Hydrieraktivität in zunehmendem Maße ab. Außerdem stellt sich eine Tendenz zur Bildung von Aldolisierungsprodukten ein.

Die Verarmung der Oberfläche des Hydrierkatalysators an Mg führt zu einer veränderten, gegenüber dem erfindungsgemäßen Katalysator ungünstigen Porenstruktur und zu einer Erhöhung der Katalysatoraktivität, die insbesondere im Hydriertemperaturbereich über 1oo °C zur Bildung unerwünschter Nebenprodukte, insbesondere Spaltprodukte führt; ein hoher Mg-Überschuß bedingt eine Inaktivierung und damit eine geringere Leistungsfähigkeit des Hydrierkatalysators.

Der Na-Anteil in der Oberflächenschicht ist für den Hydrierkatalysator zur Steuerung der Selektivität und damit zur Unterdrückung von Spalt- und Nebenreaktionen erforderlich. Erreicht wird die erhöhte Na-Konzentration in der Oberflächenschicht durch die Aufschlämmung des Grünkatalysators in Alkalilösung.

Die nachfolgenden Beispiele dienen zur Verdeutlichung der vorliegenden Erfindung, ohne sie zu begrenzen.

### Beispiel

### Herstellung des Hydrierkatalysators

19o6 g Ni (NO₃)₂ . 6 H₂O und 355,6 g Mg (NO₃)₂ . 6 H₂O werden in 1o,4 l Wasser bei 99 °C gelöst.
In einem Rührkessel werden 15oo g wasserfreies Na₂ CO₃ in 14 l Wasser bei 99 °C gelöst.

Unter kräftigem Rühren läßt man sodann die Ni-Mg-Lösung in 3 min gleichmäßig in die Soda-Lösung einlaufen. 23o g Kieselgur werden in Pulverform zugesetzt und die gebildete Suspension zunächst 3 min nachgerührt und anschließend filtriert. Der Filterkuchen wird mit 27 l 7o °C warmem Wasser gewaschen. Das letzte ablaufende Waschwasser hatte eine Leitfähigkeit von 18oo µS.
Der Filterkuchen wurde in 6ooo g 0,25 gew%iger Natronlauge suspendiert und 2 Stunden lang bei 5o °C gerührt und auf einer Filterpresse filtriert. Der Filterkuchen wird mit Preßluft 1 Minute lang behandelt. Die Feuchte des Filterkuchens betrug 82 % Wasser. In dieser Form wurde der Filterkuchen zu Fadenkorn (6 mm ⌀) verformt. Der verformte Filterkuchen wurde im Trockenschrank getrocknet, und zwar 5 Stunden lang bei 5o °C, 3 Stunden lang bei 6o °C und 8 Stunden lang bei 75 °C bis zur Gewichtskonstanz.

Der Grünkatalysator hatte folgende Analyse:

| | |
|---|---|
| Ni | 37,8 Gew% |
| MgO | 5,2 Gew% |
| Na₂O | 1,1 Gew% |
| CO₂ | 6,0 Gew% |
| Trägermaterial | 22,7 Gew% |
| Feuchte | 6,5 Gew% |
| Schüttgewicht | 53o g/l |

Der Grünkatalysator wurde bei 425 °C innerhalb von 4 Stunden reduziert. Dazu wurden über 2 kg Grünkornkatalysator 6 Nm³/h Reduktionsgas geleitet. Das Reduktionsgas bestand aus 99,5 Vol% Wasserstoff und 0,5 Vol% Stickstoff.

Bei der Reduktion wurde eine Gewichtsabnahme von 0,76 kg beobachtet.

Bei einem Gesamtnickelgehalt von 53 Gew% wurde im reduzierten Katalysator ein Reduktionsgrad von 72 % ermittelt.

### Anwendungsbeispiel 1

### Hydrierung von n-Butanal

Der nach Beispiel 1 hergestellte Katalysator wird in Form von 6 mm Tabletten (25o ml) in einem Rohrreaktor mit Heiz-Kühlmantel (Innendurchmesser: 32 mm) zunächst im Wasserstoff-Strom von 73o Nl/h bei 4 bar mit einer Aufheizgeschwindigkeit von 2o °C/h auf 12o °C gebracht. Nach Erreichen von 12o °C werden 5o ml/h n-Butanal (flüssig) in den, dem Reaktor vorgeschalteten, von 73o Nl/h H₂ durchströmten Verdampfer bei 1oo °C Verdampfertemperatur eingespeist. Das H₂/Butanal-Dampfgemisch wird vor dem Reaktor in einem Vorerhitzer auf Reaktortemperatur aufgeheizt. Nach 12 h wird die Aufgabe-Menge an n-Butanal auf 75 ml/h und nach weiteren 12 h auf 1oo ml/h erhöht. Danach erfolgt die Steigerung der Butanal-Aufgabe jeweils in Raten von 25 ml n-Butanal im 24 Stunden-Intervall. Gleichzeitig wird im Verlauf der Butanal-Aufgabe-Steigerung die Vorerhitzor- und Reaktortemperatur wie folgt erhöht:

| n-Butanal-Aufgabe (ml/h) | Zeit (h) | Vorerhitzer/Reaktionstemperatur (°C) |
|---|---|---|
| 200 | 24 | 123 |
| 225 | 24 | 126 |
| 250 | Dauerbetrieb | 128 |

Die H₂-Menge wird während der Anfahrperiode und während des Dauerbetriebes konstant gehalten.
Das dampfförmig anfallende Hydrierprodukt wird kondensiert und analysiert.
Das Hydrierprodukt besteht zu 99,9 Gew% aus n-Butanol und enthält weniger als 0,1 Gew% an nicht umgesetztem n-Butanal sowie < 20 ppm Di-n-Butylether als Nebenprodukt.
Neben dem Hydrierprodukt werden ca. 0,4 kg H₂O-Dampf im Dauerbetrieb von > 1,8 bar (Dampfdruck entsprechend der Reaktortemperatur) pro kg eingesetztes n-Butanal gewonnen.

### Anwendungsbeispiel 2

### Hydrierung von Propanal

Der nach Beispiel 1 hergestellte Katalysator wird in Form von 6 mm-Tabletten (250 ml) in dem gleichen Reaktorsystem wie im Anwendungsbeispiel 1 zunächst im H₂-Strom (730 Nl/h) bei 3,5 bar mit einer Aufheizgeschwindigkeit von 20°C/h auf 125°C aufgeheizt. Danach werden über einen Zeitraum von 12 h zunächst 50 ml/h Propanal (flüssig) in den Verdampfer eingespeist. Die eingespeiste Propanal-Menge wird im Zeitabstand von jeweils 12 h um je 25 ml/h bis auf 150 ml/h gesteigert. Bei Erreichen der Aufgabemenge von 150 ml/h wird die Vorerhitzer- und Reaktortemperatur auf 128 bis 130°C erhöht. Das anfallende Hydrierprodukt wird durch Kühlung kondensiert und analysiert. Es besteht zu ca. 99,9 Gew% aus n-Propanol und
< 0,1 Gew% nicht umgesetztem Propanal. Als Nebenprodukte werden < 100 ppm 2-Methylpentanon-3 und < 20 ppm Di-n-Propylether analysiert.
Wie in Anwendungsbeispiel 1 ist auch bei der Hydrierung von Propanal mit dem erfindungsgemäßen Katalysator die Gewinnung von Wasserdampf (≧ 1,3 bar) möglich.

### Anwendungsbeispiel 3

### Hydrierung von i-Butanal

Für die Hydrierung von i-Butanal wurde der Katalysator (250 ml) aus Beispiel 1 unter den gleichen Bedingungen wie im Anwendungsbeispiel 1 unter H₂ auf 120 °C aufgeheizt. Anschließend wurden 50 ml/h i-Butanal eingespeist. Hydrierdruck (3,5 bar), Reaktortemperatur (120 °C) und H₂-Aufgabegeschwindigkeit (730 Nl/h) wurden konstant gehalten.
Das anfallende Hydrierprodukt bestand zu ≧ 99,95 Gew% aus i-Butanol und enthielt ≦ 0,03 Gew% nichtumgesetztes i-Butanal und < 20 ppm Di-i-Butylether. Die Hydrierung von i-Butanal an dem erfindungsgemäßen Katalysator liefert H₂O-Dampf von ≧ 1,8 bar.

### Vergleichsbeispiel

### Hydrierung von n-Butanal

In dem gleichen Rohrreaktor wie im Anwendungsbeispiel 1 werden 250 ml des kommerziellen Nickelkatalysators 55/5 TST (HOECHST AG) in Form von 6 mm-Tabletten im Wasserstoffstrom von 730 Nl/h bei 3,5 bar mit einer Aufheizgeschwindigkeit von 20 °C/h auf 100 °C aufgeheizt.
Danach wurden 50 ml/h n-Butanal (flüssig) in den, dem Reaktor vorgeschalteten, von 730 Nl/h H₂ durchströmten Verdampfer bei 1oo °C eingespeist. Vor Eintritt in den Reaktor wird das H₂-Butanal-Dampfgemisch in einem Vorerhitzer auf Reaktortemperatur aufgeheizt.
Nach 12 h wird die aufgegebene Menge n-Butanal auf 75 ml/h und in Intervallen von jeweils 12 h bis auf 15o ml/h gesteigert. Unter diesen Reaktionsbedingungen wurde ein Hydrierprodukt erhalten, welches aus 98,9 Gew% n-Butanol, 0,3 Gew% Acetalen, 0,3 Gew% trimeren Aldolisierungsprodukten des Butanals, 0,1 Gew% Kohlenwasserstoffen, 0,1 Gew% 2-Ethylhexanol, 0,1 Gew% Di-n- butylether und 2oo bis 3oo ppm C₄/C₄-Ester bestand.
Vom eingesetzten n-Butanal wurden 1,0 bis 1,2 Gew% durch Hydrogenolyse zu Propan und Methan umgewandelt, die im abströmenden Hydrierwasserstoff enthalten sind.
Gegenüber dem erfindungsgemäßen Katalysator treten bei Verwendung des handelsüblichen Katalysators bereits bei 1oo °C erhebliche Anteile an Nebenprodukten auf. Besonders schwierig ist für die Gewinnung von reinem n-Butanol die destillative Abtrennung des Di-n-Butylethers, welche mit steigenden Estergehalten zu überproportionalen Verlusten an n-Butanol führt.
Weiterhin nachteilig ist gegenüber dem erfindungsgemäßen Katalysator der durch Hydrogenolyse verursachte, relativ hohe Verlust von Wertprodukt (> 1 Gew%).

Wird die Aufgabe von n-Butanal über 15o ml/h gesteigert, so steigt insbesondere die Bildung von Di-n-Butyl-Ether und Spaltprodukten durch Hydrogenolyse an.
Gleiche Effekte stellen sich bei Erhöhung der Hydriertemperatur ein.
Im Vergleich zum erfindungsgemäßen Katalysator können mit dem Katalysator des Standes der Technik maximal nur 6o % der Aldehydmenge umgesetzt werden, wobei außerdem stärker verunreinigte Hydrierprodukte erhalten werden.

Als weiterer Nachteil ist die Begrenzung der Hydriertemperatur auf maximal 100°C anzusehen, wobei kein nutzbarer Wasserdampf erhalten wird.

## Patentansprüche

1. Hydrierkatalysator enthaltend Nickel, Nickeloxid, Magnesiumoxid, Natriumoxid und ein wasserunlösliches Trägermaterial, dadurch gekennzeichnet, daß der Hydrierkatalysator aus
25 bis 5o Gewichts% Nickel (metallisch)
1o bis 35 Gewichts% Nickeloxid
4 bis 12 Gewichts% Magnesiumoxid
1 bis 5 Gewichts% Natriumoxid
Rest Trägermaterial
besteht und die Summe aus Nickel und Nickeloxid 4o bis 7o Gewichts% beträgt, wobei der Hydrierkatalysator eine, nach BET bestimmte, Oberfläche von 8o bis 2oo m²/g und ein, durch Hg-Porosimetrie bestimmtes, Gesamtporenvolumen von 0,35 bis 0,6 ml/g hat, und das Gesamtporenvolumen aufgeteilt ist in
3o bis 6o Volumenprozenten mit einem Porenradius von ≦ 4 nm (4o Å),
4 bis 1o Volumenprozenten mit einem Porenradius von > 4 bis 30 nm (4o bis 3oo Å),
3o bis 6o Volumenprozenten mit einem Porenradius von > 30 bis 500 nm (3oo bis 5ooo Å).

2. Hydrierkatalysator nach Anspruch 1, dadurch gekennzeichnet, daß 5-10 Atomlagen auf der Oberfläche des Hydrierkatalysators, bestimmt nach der SAM-Untersuchung,
18 bis 30, vorzugsweise 20 bis 28 Atom% Ni
1,2 bis 3,0, vorzugsweise 1,5 bis 2,5 Atom% Na
2,8 bis 4,8, vorzugsweise 3,2 bis 4,5 Atom% Mg
enthalten.

3. Hydrierkatalysator nach einem der Ansprüche 1 und 2, dadurch gekennzeichnet, daß die metallische Nickeloberfläche eine, durch Chemiesorption von Wasserstoff bestimmte, Größe von 100 bis 130 m²/g Ni hat.

4. Hydrierkatalysator nach den Ansprüchen 1 bis 3, dadurch gekennzeichnet, daß er als Trägermaterial Aluminiumoxid oder Siliziumdioxid, insbesondere in Form von Kieselsäure, Kieselgel, Kieselgur oder Kieselerde enthält.

5. Verfahren zur Herstellung des Hydrierkatalysators nach mindestens einem der Ansprüche 1 bis 4, dadurch gekennzeichnet, daß man in einer Fällstufe einen Grünkatalysator aus Nickelsalz, Magnesiumsalz und Soda sowie Trägermaterial herstellt, diesen Grünkatalysator nach Abtrennen der Mutterlauge und partieller Waschung in Alkalilösung aufschlämmt, von der Flüssigphase abtrennt, trocknet und den getrockneten Grünkatalysator mit Wasserstoff behandelt, bis 42 bis 83 Gew% des gesamten Nickelanteils in metallischer Form vorliegen.

6. Verfahren nach Anspruch 5, dadurch gekennzeichnet, daß man in der Fällstufe in eine 95 bis 100°C heiße 0,9 bis 1,1 molare Sodalösung eine 95 bis 100°C heiße wäßrige Lösung, enthaltend 0,5 bis 0,8 mol/l Nickelsalz und 0,1 bis 0,2 mol/l Magnesiumsalz, bis zu einem molaren Verhältnis von Na₂CO₃ : (Ni + Mg) = 1 : 0,60 bis 0,65 einrührt und anschließend umgehend innerhalb von 0,5 bis 5 Minuten das Trägermaterial zusetzt und den gebildeten Grünkatalysator von der Mutterlauge abfiltriert, mit heißem Wasser partiell wäscht, bis das ablaufende Waschwasser eine Leitfähigkeit von 1500 bis 2000 µS hat; danach den Grünkatalysator in einer 1- bis 3-fachen Wassermenge suspendiert und mit 0,06 bis 0,08 Mol Natronlauge oder 0,03 bis 0,04 Mol Soda pro Mol in der Fällstufe eingesetzten Nickelmenge verrührt und nach 1 bis 5 Stunden Rührzeit bei einer Temperatur von 40 bis 60°C den Grünkatalysator aus der Suspension abtrennt.

7. Verfahren nach einem der Ansprüche 5 bis 6, dadurch gekennzeichnet, daß man den aus der Suspension abgetrennten und getrockneten Grünkatalysator bei einer Temperatur von 350 bis 450°C reduziert, indem man den getrockneten Grünkatalysator mit 0,5 bis 5,0 Nm³/h Reduktionsgas pro kg Grünkatalysator, wobei das Reduktionsgas 80 bis 100 Vol% Wasserstoff enthält, behandelt.

8. Verfahren nach Anspruch 7, dadurch gekennzeichnet, daß man 1 bis 3 Nm³/h Reduktionsgas pro kg getrocknetem Grünkatalysator einsetzt.

9. Verwendung des Hydrierkatalysators nach mindestens einem der Ansprüche 1 bis 4 zur Hydrierung von Propanal, n-Butanal und i-Butanal.

10. Verwendung des Hydrierkatalysators nach Anspruch 9, dadurch gekennzeichnet, daß man den Hydrierkatalysator bei 1oo bis 16o °C einsetzt.

## Claims

1. A hydrogenation catalyst comprising nickel, nickel oxide, magnesium oxide, sodium oxide and a water-insoluble support material, said hydrogenation catalyst containing
from 25 to 50 % by weight of nickel (metallic)
from 10 to 35 % by weight of nickel oxide
from 4 to 12 % by weight of magnesium oxide
from 1 to 5 % by weight of sodium oxide
remainder support material,
where the total of nickel and nickel oxide is from 40 to 70% by weight, and having a surface area determined by BET from 80 to 200 m²/g and a total pore volume determined by Hg porosimetry from 0.35 to 0.6 ml/g, with the total pore volume being made up of
from 30 to 60 percent by volume having a pore radius of ≦ 4 nm (40 Å),
from 4 to 10 percent by volume having a pore radius of > 4 to 30 nm (40 to 300 Å),
from 30 to 60 percent by volume having a pore radius of > 30 to 500 nm (300 to 5000 Å).

2. A hydrogenation catalyst as claimed in claim 1, wherein 5-10 atomic layers on the surface of the hydrogenation catalyst contain, determined by SAM analysis,
from 18 to 30, preferably from 20 to 28, atom% Ni
from 1.2 to 3.0, preferably from 1.5 to 2.5, atom% Na
from 2.8 to 4.8, preferably from 3.2 to 4.5, atom% Mg.

3. A hydrogenation catalyst as claimed in either of claims 1 and 2, wherein the metallic nickel surface area is, as determined by chemisorption of hydrogen, from 100 to 130 m²/g Ni.

4. A hydrogenation catalyst as claimed in any of claims 1 to 3, which contains aluminum oxide or silicon dioxide, particularly in the form of silicic acid, silica gel, kieselguhr or siliceous earth, as support material.

5. A process for preparing the hydrogenation catalyst as claimed in at least one of claims 1 to 4, which comprises preparing, in a precipitation step, a green catalyst from nickel salt, magnesium salt and sodium carbonate and also a support material, slurrying this green catalyst, after separating off the mother liquor and partial washing, in alkali solution, separating it from the liquid phase, drying and treating the dried green catalyst with hydrogen until from 42 to 83 % by weight of the total nickel content is present in metallic form.

6. The process as claimed in claim 5, wherein, in the precipitation step, an aqueous solution containing from 0.5 to 0.8 mol/l of nickel salt and 0.1 to 0.2 mol/l of magnesium salt and at a temperature from 95 to 100°C is stirred into a from 0.9 to 1.1 molar sodium carbonate solution at a temperature from 95 to 100°C until the molar ratio of Na₂CO₃ : (Ni + Mg) = 1 : from 0.60 to 0.65 and subsequently the support material is immediately added over a period of from 0.5 to 5 minutes and the green catalyst formed is filtered off from the mother liquor and partially washed with hot water, until the wash water which runs off has a conductivity from 1500 to 2000 µS; the green catalyst is then suspended in from 1 to 3 times the amount of water and admixed by stirring with from 0.06 to 0.08 mol of sodium hydroxide solution or from 0.03 to 0.04 mol of sodium carbonate per mole of nickel used in the precipitation step, and after from 1 to 5 hours' stirring at a temperature from 40 to 60°C the green catalyst is separated off from the suspension.

7. The process as claimed in either of claims 5 and 6, wherein the green catalyst which has been separated off from the suspension and dried is reduced at a temperature from 350 to 450°C by treating the dried green catalyst with from 0.5 to 5.0 standard m³/h of reduction gas per kg of green catalyst, the reduction gas containing from 80 to 100% by volume of hydrogen.

8. The process as claimed in claim 7, wherein from 1 to 3 standard m³/h of reduction gas are used per kg of dried green catalyst.

9. A method of using the hydrogenation catalyst in at least one of claims 1 to 4 for the hydrogenation of propanal, n-butanal and i-butanal.

10. A method of using the hydrogenation catalyst as claimed in claim 9, wherein the hydrogenation catalyst is used at from 100 to 160°C.

## Revendications

1. Catalyseur d'hydrogénation contenant du nickel, de l'oxyde de magnésium, de l'oxyde de sodium et une matière de support insoluble dans l'eau, caractérisé en ce qu'il consiste en :
- 25 à 50 % en poids de nickel (métallique),
- 10 à 35 % en poids d'oxyde de nickel,
- 4 à 12 % en poids d'oxyde de magnésium,
- 1 à 5 % en poids d'oxyde de sodium,
complément : matière de support,
et la somme des teneurs en nickel et en oxyde de nickel représente de 40 à 70 % en poids, ce catalyseur d'hydrogénation ayant une surface BET de 80 à 200 m²/g, un volume poreux total, déterminé par porosimétrie au mercure, de 0,35 à 0,6 ml/g, avec la répartition suivante du volume poreux total :
- 30 à 60 % en volume des pores ont un rayon ≦ 4 nm,
- 4 à 10 % en volume des porcs ont un rayon > 4 à 30 nm,
- 30 à 60 % en volume des porcs ont un rayon > 30 à 500 nm

2. Catalyseur d'hydrogénation selon la revendication 1, caractérisé en ce que 5 à 10 positions atomiques à la surface du catalyseur contiennent, à l'examen par spectroscopie SAM,
18 à 30, de préférence 20 à 28, atomes % de Ni,
1,2 à 3,0, de préférence 1,5 à 2,5, atomes % de Na,
2,8 à 4,8, de préférence 3,2 à 4,5 atomes % de Mg;

3. Catalyseur d'hydrogénation selon l'une des revendications 1 et 2, caractérisé en ce que la surface de nickel métallique a une dimension, déterminée par chimisorption d'hydrogène, de 100 à 130 m²/g de nickel.

4. Catalyseur d'hydrogénation selon les revendications 1 à 3, caractérisé en ce qu'il contient, en tant que matière de support, de l'alumine ou de la silice, plus spécialement sous la forme d'acide silicique, de gel de silice, de kieselguhr ou d'alumine naturelle.

5. Procédé de préparation du catalyseur d'hydrogénation selon au moins une des revendications 1 à 4, caractérisé en ce que, dans une opération de précipitation, on prépare un catalyseur cru à partir d'un sel de nickel, d'un sel de magnésium, de carbonate de sodium, et d'une matière de support, on sépare les liqueurs mères et on lave partiellement le catalyseur cru, on le disperse dans une solution alcaline, on le sépare de la phase liquide, on le sèche, puis on le traite par l'hydrogène jusqu'à ce que 42 à 83 % du poids total du nickel soit à l'état métallique.

6. Procédé selon la revendication 5, caractérisé en ce que, à l'opération de précipitation, on introduit sous agitation une solution aqueuse portée à 95-100°C, contenant 0,5 à 0,8 mole/l de sel de nickel et 0,1 à 0,2 mole/l de sel de magnésium dans une solution de carbonate de sodium 0,9 à 1,1 M chauffée à 95-100°C, jusqu'à un rapport molaire Na₂CO₃/(Ni + Mg) = 1 : 0,60 à 0,65, puis, immédiatement, dans un délai de 0,5 à 5 minutes, on ajoute la matière de support et on sépare le catalyseur cru ainsi formé des liqueurs mères par filtration, on le lave partiellement à l'eau chaude jusqu'à ce que l'eau de lavage ait une conductivité de 1500 à 2000 µS ; on met le catalyseur cru en suspension dans une à trois fois son poids d'eau et on mélange sous agitation avec 0,06 à 0,08 mole de lessive de soude ou 0,03 à 0,04 mole de carbonate de sodium par mole de la quantité de nickel mise en oeuvre à la précipitation et après 1 à 5 h d'agitation à une température de 40 à 60°C, on sépare le catalyseur cru de la suspension.

7. Procédé selon l'une des revendications 5 et 6, caractérisé en ce que l'on réduit, à une température de 350 à 450°C, le catalyseur cru séparé de la suspension et séché, en le traitant par 0,5 à 5,0 m³ normaux/h d'un gaz réducteur par kg du catalyseur cru, ce gaz réducteur contenant de 80 à 100 % en volume d'hydrogène.

8. Procédé selon la revendication 7, caractérisé en ce que l'on utilise de 1 à 3 m³ normaux/h de gaz réducteur par kg de catalyseur cru séché.

9. Utilisation du catalyseur d'hydrogénation selon au moins une des revendications 1 à 4 pour l'hydrogénation du propanal, du n-butanal et de l'isobutanal.

10. Utilisation du catalyseur d'hydrogénation selon la revendication 9, caractérisé en ce qu'on le met en oeuvre à une température de 100 à 160°C.
